Europäisches Patentamt

European Patent Office

Office européen des brevets

(19)

(11) Publication number: **0 134 855**
**B1**

## (12) EUROPEAN PATENT SPECIFICATION

(45) Date of publication of patent specification: **23.12.87**

(51) Int. Cl.⁴: **A 01 G 33/00, A 01 K 61/00**

(21) Application number: **83305434.9**

(22) Date of filing: **15.09.83**

(54) **Concrete blocks for use underwater for algal culture.**

(43) Date of publication of application:
**27.03.85 Bulletin 85/13**

(45) Publication of the grant of the patent:
**23.12.87 Bulletin 87/52**

(84) Designated Contracting States:
**FR GB IT**

(56) References cited:
**FR-A-2 199 438**
**US-A-4 165 711**

**PETROLEUM ENGINEER INTERNATIONAL, vol.
54, no. 6, May 1982, Dallas D.J. SHEEHY et al.
"Artificial reefs - a second life for offshore
platforms?, pages 40, 42, 44, 48, 50, 52**
**Patent Abstracts of Japan vol. 7, no. 86, 9 April
1983**

(73) Proprietor: **Hokuriku Estate Co., Ltd**
**4-6-15, Matsumoto,**
**Fukui-shi, Fukui 910 (JP)**

(72) Inventor: **Suzuki, Tetsuo**
**12-6, Tsutsujigaoka-cho**
**Sabae-shi Fukui (JP)**

(74) Representative: **Diamond, Bryan Clive et al**
**Gee & Co. Chancery House Chancery Lane**
**London WC2A 1QU (GB)**

# Description

This invention relates to an algal culturing reef unit, an artificial reef unit and an artificial culturing and fishing area or field unit. More particularly, it relates to such units adapted for raising algae necessary for spawning and hatching of fishery products such as fishes, shellfishes and crustacean and culturing and protection of the young fishes, shellfishes and crustacean, and adapted for providing an environment suitable for raising these fishery products.

Recently, various artificial reef units have been developed and practiced for protection and raising of fishery products such as fishes and shellfishes see e.g. US—A—4 165 711. These units are intended for attracting adult fishes and shellfishes for providing an artificial fishing field and are also termed as swarming reef. This type of reef may be produced by submerging used vessels, used cars, waste tyres or concrete, steel, stone or synthetic resin materials assembled or molded into boxes or lattices.

On account of the recent tendency towards depletion of fishery resources and the problem of the 200-sea mile territory, there is an increasing demand for developing shore fishing. This has led to the development of the artificial reef unit aimed at raising or culturing fishery resources besides the aforementioned swarming reef. This culturing type reef is intended for protection and raising of larval and juvenile fishes and shellfishes.

For raising these juvenile marine animals, it is necessary to provide an environment suitable for settlement and growth of diatoms and other feed plants. Thus, in distinction from the swarming type reef, the culturing reef need to be submerged in a shallow sea zone with a depth between 5 and 15 meters where there is sufficient supply of natural light beams. However, because of high waves and fast oceanic currents usually encountered in such sea zone, the culturing type reef is required to be sufficiently resistant to these severe environments.

Thus, the fishes and shellfishes would swim in water for a certain period after hatching and grow in size grazing on diatoms or other plants or microorganisms. The fishes and shellfishes are called juvenile in this stage of development. Thus the presence of diatoms and microorganisms is imperative for the growth of these young aquatic animals. For growth of diatom, it is essential to supply a sufficient quantity of oxygen and sunbeams. To this end, the culturing reef unit must be submerged in a shallow sea zone subject to adverse environments and hence must be arranged and constructed to withstand the severe operating environments.

From economic considerations, the reef unit is preferably made of concrete in order to resist the adverse conditions usually met in the shallow sea zone. However, when submerged in sea water, concrete units or blocks emit a strong alkali from their surface (pH value, about 13). This strong alkali thus emitted into the sea water acts to hinder the settlement or growth of green algae or diatoms or other microorganisms and may even kill them. When extinct, these algae are converted into a layer of calcares known as arthrocalcares, this layer building up on the concrete surface and interfering with settlement and habitation of the algae. In order to prevent this, it is necessary to neutralize the strong alkali emitted from the concrete surface. However, there has not yet been known an effective means of neutralization.

In addition, the basic culturing conditions are different from one aquatic animal to another because of difference in their habit, feed and manner of living. However, these basic culturing conditions have not been sufficiently considered for the known types of artificial reefs units or culturing and fishing reef units.

According to the present invention we provide a concrete block suitable for use underwater as a reef for the growth of algae, characterised in that the block is provided on its surface with an iron sulfate in a form which neutralises alkali leached from the concrete.

We furthermore provide an artificial culturing and fishing unit comprising a combination of the different types of said concrete blocks or assemblies suitable for the growth of fish or edible underwater animals of different types. These units function as artificial reefs; and they meet the aforementioned culture requirements since the main body of the artificial culturing unit is made of concrete in order to be resistant to high waves and fast oceanic current proper to the shallow zone in a manner so that the strong alkkali emitted from the surface of the concrete block is effectively neutralized and iron oxide contents necessary for growth of algae and fishes or shellfishes are caused to exist in abundance on the surface of the concrete block.

In the manufacture of a concrete block of the present invention, crystal blocks of iron sulfate or iron (II) sulfate are embedded below the surface of the concrete base whilst the concrete is not yet cured. As the concrete is hardened, the crystal blocks are dissolved in the moisture formed from the concrete so that a porous layer of iron sulfate or iron (II) sulfate is formed on the resulting recesses on the concrete surface, said layer penetrating into the inside of the concrete, and the strong alkali contained in the concrete being neutralized with the acid from the iron sulfate or iron (II) sulfate. Alternatively, after the concrete base block has hardened, a solvent consisting of a mixture of a small amount of acid such as sulfuric acid and iron oxide powders such as iron sulfate or iron (II) sulfate powders and a concrete penetrant in turn consisting of a surfactant is coated on the surface of the base block for forming a layer of iron oxide powders such as iron sulfate or iron (II) sulfate powders, said layer penetrating into the inside of the concrete base block, the acid of the iron sulfate or iron (II) sulfate or that contained in the solvent acting for neutralizing the concrete alkali. According to the invention, this technology is utilized for providing an artificial alga culturing unit, an artificial reef unit and an artificial culturing and fishing field unit.

Fig. 1 is a perspective view showing a square portion of a base plate used in the algal culturing unit of the present invention.

Figs. 2 and 3 show steps in its manufacture.

Fig. 4 is an enlarged view showing a portion A shown in Fig. 3.

Fig. 5 is a perspective view showing a square portion of another embodiment of a base portion used in the algal culturing unit of the present invention.

Fig. 6 is an enlarged view showing a portion B shown in Fig. 5.

Fig. 7 is a perspective view showing an example of an artificial algal culturing reef unit of the present invention.

Figs. 8 to 10 illustrate an example of an artificial reef unit according to the present invention, wherein

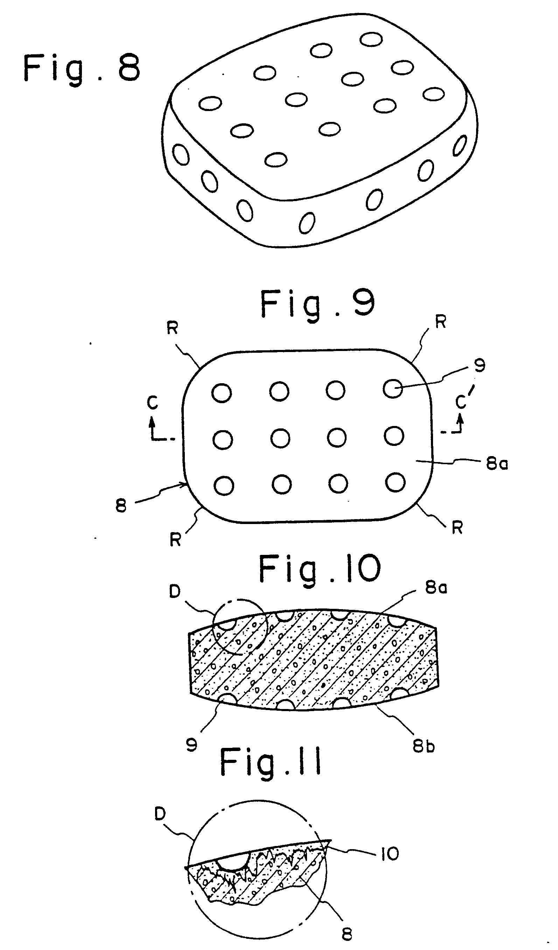

Fig. 8 is an overall perspective view of the reef unit.

Fig. 9 is a plan view of the reef unit.

Fig. 10 is a sectional view taken along line C—C' of Fig. 9.

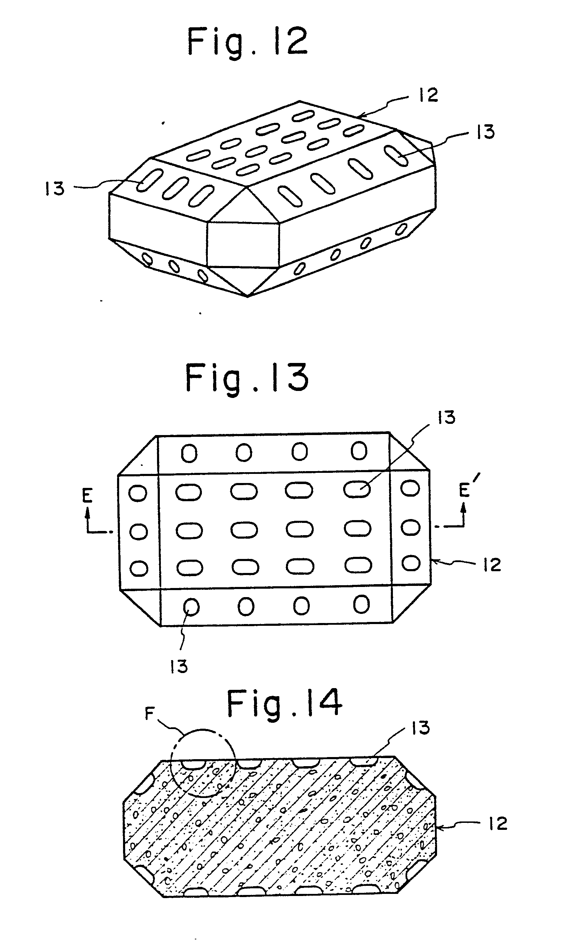

Fig. 11 is an enlarged view showing a portion D shown in Fig. 10.

Figs. 12 to 14 illustrate a modification of the artificial reef unit according to the present invention, wherein

Fig. 12 is an overall perspective view of the reef unit.

Fig. 13 is a plan view of the reef unit.

Fig. 14 is a sectional view taken along line E—E' of Fig. 13.

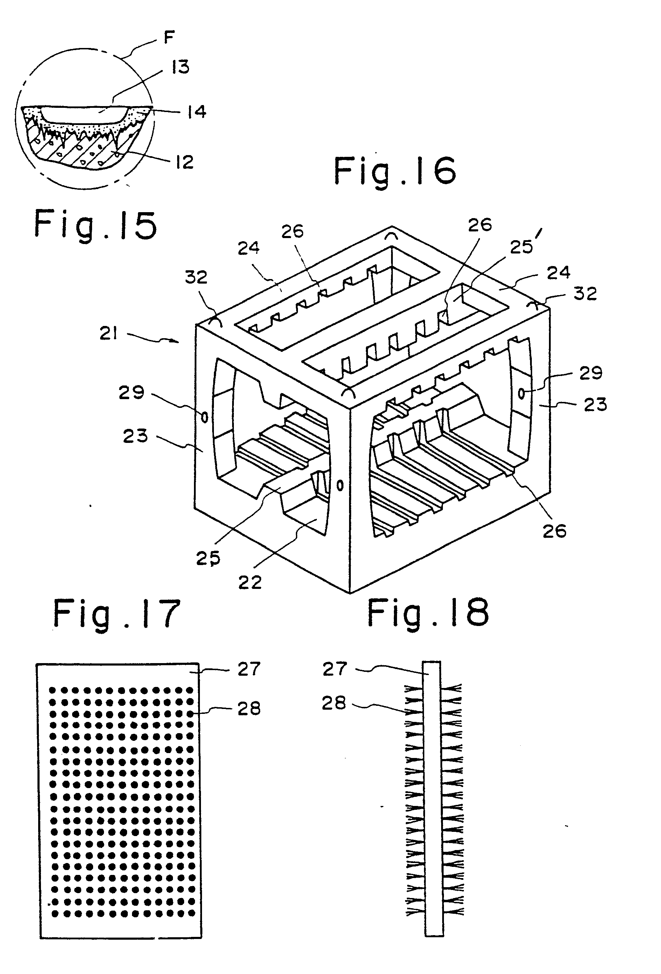

Fig. 15 is an enlarged view of a portion F in Fig. 14.

Figs. 16 to 19 illustrate a settlement reef unit adapted for settlement of larval and juvenile fish and shellfish, wherein

Fig. 16 is a perspective view of a reef frame.

Figs. 17 and 18 are front and side views of a flocked plate, respectively.

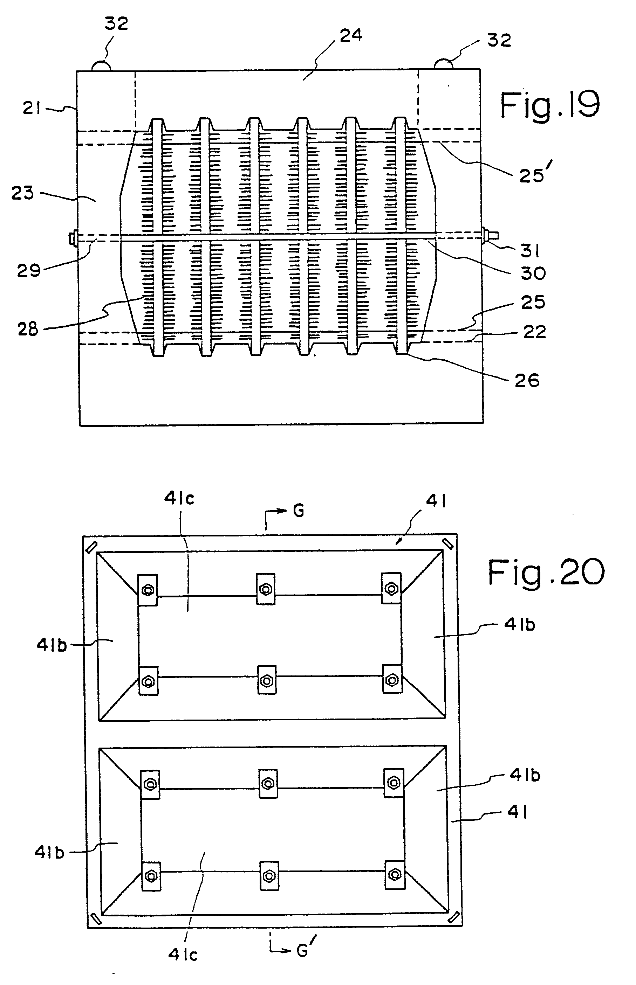

Fig. 19 is a front view of the settlement reef unit.

Figs. 20 to 22 illustrate a raising reef unit aimed at raising fish and shellfish, especially sea crayfish wherein

Fig. 20 is a plan view of the reef unit.

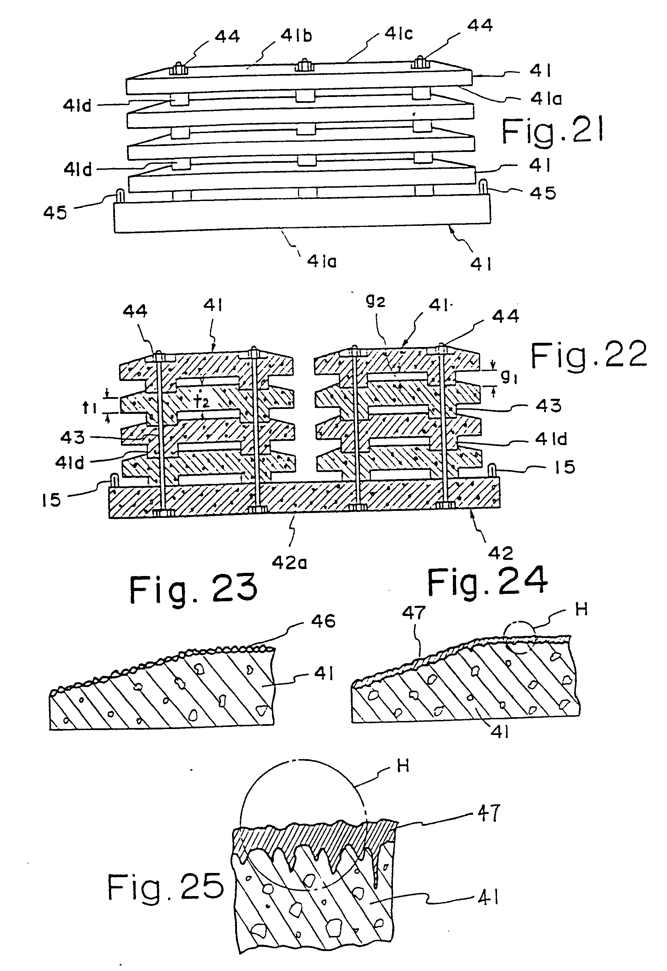

Fig. 21 is a front view of the reef unit.

Fig. 22 is a sectional view taken along line G—G of Fig. 20.

Figs. 23 to 27 are partial sectional views showing the manufacture of the raising unit block.

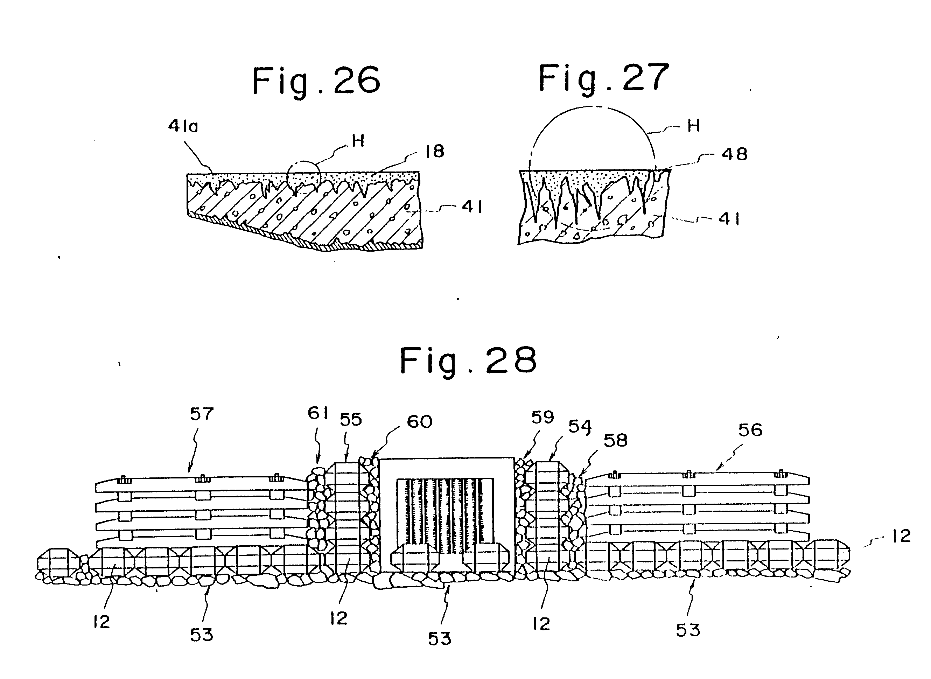

Figs. 28 to 30 illustrate a culturing and fishing field unit for culturing fish and shellfish, especially sea crayfish, wherein

Fig. 28 is a front view of the field unit.

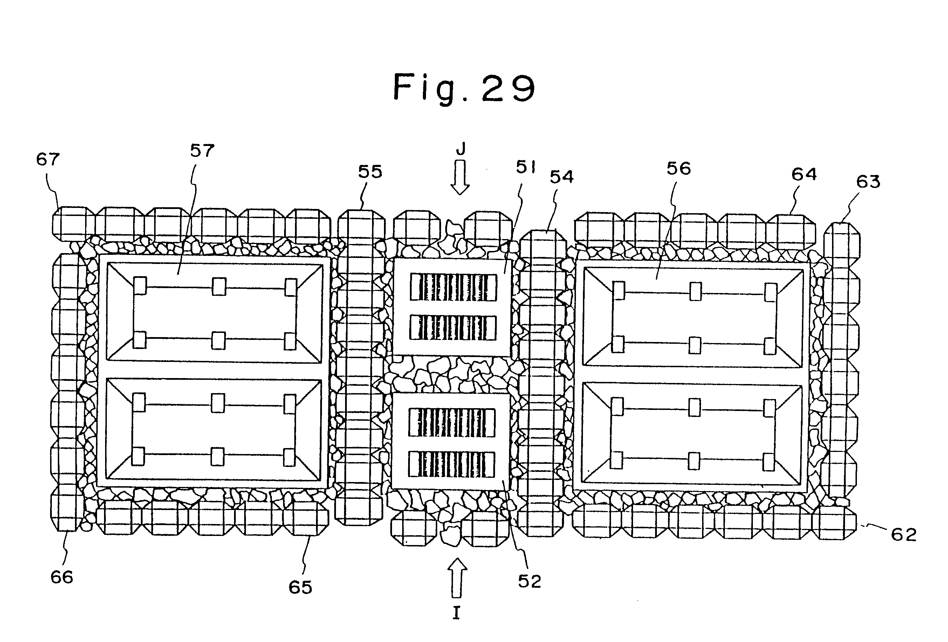

Fig. 29 is a plan view of the field unit.

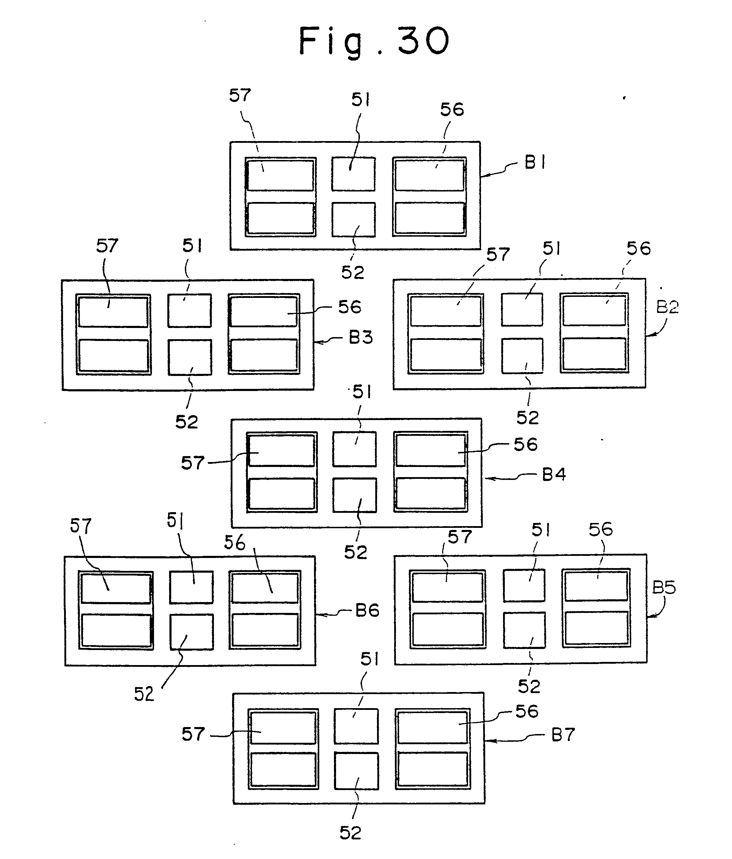

Fig. 30 is a schematic view of a culturing and fishing field unit combined from a plurality of fishing field units shown in Figs. 28, 29.

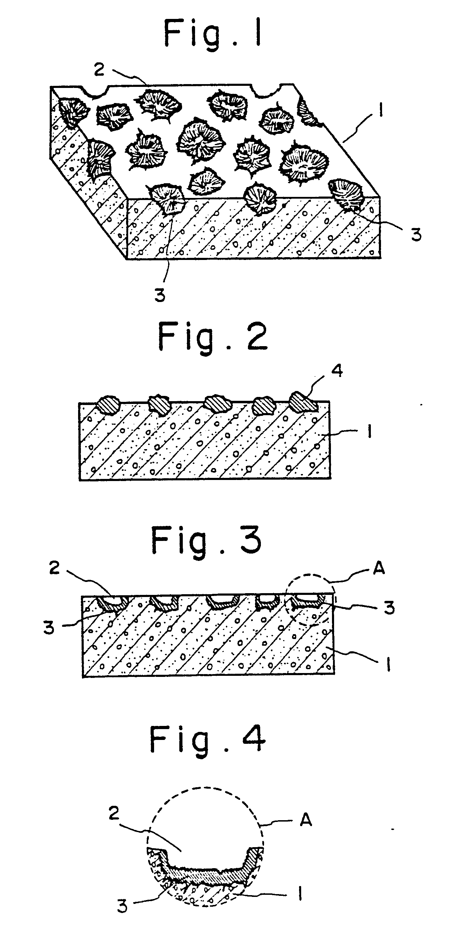

Fig. 1 shows in perspective a substantially square-shaped portion cut from a base plate used in an algal culturing reef unit embodying the present invention. Numeral 1 designates a concrete base plate. Crystalline blocks of iron sulfate or iron (II) sulfate are embedded below the surface of the base plate whilst the concrete is as yet not cured. As the concrete is hardened through curing, the crystals are dissolved in the water of the concrete for forming a number of pits or recesses 2 on the base plate surface. A layer 3 of iron sulfate or iron (II) sulfate is formed on the surface of the recess 2.

Figs. 2 and 3 show in partial section the manufacture process of the aforementioned algal culturing base plate.

The base plate 1 is cast by pouring mortar into a framework of any desired shape or structure, not shown. While the concrete of the base plate 1 is not hardened, a number of small-sized blocks 4 of iron sulfate or iron (II) sulfate are embedded, at least partially, below the base plate surface, as shown in Fig. 2, and the base plate 1 is cured in this state. During this curing stage, the concrete of the base plate 1 is dehydrated and hardened. On the other hand, the blocks 4 are dissolved in water yielded from the concrete for forming pits or recesses 2 shown in Fig. 3. On the surfaces of these recesses 2, there are formed porous layers 3 of iron (III) sulfate or iron (II) sulfate, these layers 3 having extremely irregular upper surfaces as shown in Fig. 4 and reverse surfaces in contact with and penetrating into the inside of the concrete like the roots of the tree so as to resist peeling of the porous layers from the concrete surface.

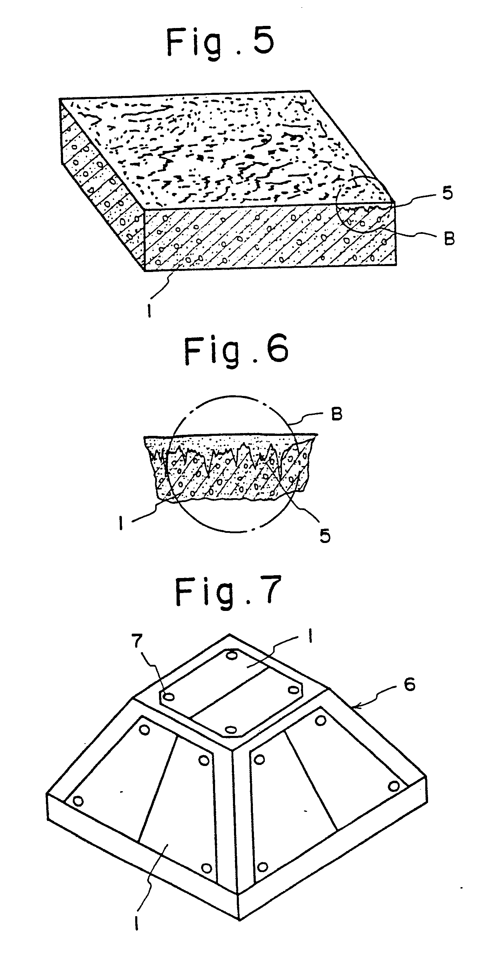

in Fig. 5, a square piece of the base plate used in an algal culturing reef according to another embodiment of the present invention is shown in perspective. A solvent prepared by mixing comminuted particles of iron sulfate or iron (II) sulfate into a liquid concrete penetrant consisting of any suitable surface active agent is coated on the upper surface of the base plate 1. The iron sulfate or iron (II) sulfate particles are penetrated from the surface into the inside of the concrete base plate due to the action of the surfactant. As shown in Fig. 6, which is a detailed sectional view showing a portion surrounded in circle in Fig. 5, the iron sulfate or iron (II) sulfate particles are penetrated from the base plate surface like the tree roots, due to the action of the surfactant, thus providing a penetrated layer 5. These iron sulfate or iron II sulfate particles are preferably small in size so as to be penetratable into the concrete due to the assistive function of the surfactant. It has been found that a depth of penetration which is larger than 5 cm from the surface into the inside of the concrete may be realized with the particle size of 0.8 to 1 μm.

Alternatively, a solvent consisting of the concrete penetrant or surfactant and a mixture of fine particles of iron oxide less than 1 μm in size with a small amount of acid such as sulfuric acid may be used for providing the penetrated layer 5 consisting of the acid and the particles of iron oxide.

The penetrated layer 3 of iron sulfate or iron (II) sulfate on the base plate shown in Figs. 1 and 5 is strongly acidic because of acid contents in the solvent or the iron sulfate and is capable of neutralizing the alkaline contents in the concrete of the base plate 1. Hence, when the aforementioned base plates are placed on the shallow sea floor either singly or as components of a variety of structural units, the concrete surface portions covered by the penetrated layers 3 or 5 are not exposed to sea water so that any alkaline contents emanating from the concrete are neutralized by the acid mixed in the solvent or that contained in iron sulfate or iron (II) sulfate and thus prevented from going into sea water. In addition, since there is great abundance of the iron oxides which are liked by algae and marine organisms, there is provided an environment highly suitable for settlement and growth of sea plants grazed by the marine organisms, such as algae or green algae.

Fig. 7 shows an algal culturing reef unit consisting of a concrete block 6 in the form of a frustrum of a regular pyramid on each surface of which a piece of the base plate 1 shown in Figs. 1 or 5 cut into a corresponding profile is secured with suitable fasteners 7 such as bolts and nuts. This reef unit is placed on the shallow sea bottom 5 to 10 meters deep where high waves or currents are encountered. The waves or currents striking the lateral sides of the block 6 flow upwards along the inclined surfaces of the block 6 thus giving rise to an upward turbulent flow of sea water. On account of such turbulent flow of sea water, there may be assured an abundant supply of oxygen and sunlight necessary for survival and growth of algae and aquatic organisms.

The applicant has conducted an experiment with the aforementioned algal culturing reef unit placed on the shallow sea bottom of the Japan Sea at a depth of 5 to 10 meters. It has been found that numerous sea plants such as green alga and kurome were settled on the blocks in the winter six-month period of November to April and that shellfish such as abalone and top shells also settled on the blocks.

Fig. 8 is an overall perspective view of an artificial reef embodying the present invention and Fig. 9 is a plan view thereof. As shown in Fig. 10, which is a section taken along line C—C' in Fig. 9, the four corners of a rectangular concrete block 8 are rounded at R, and the convex upper and lower surfaces 8a, 8b as well as lateral surfaces 8c of the block are provided with a number of hemispherical pits or recesses 9. The surfaces of the concrete block 8 are coated with a solvent prepared by mixing fine particles of iron sulfate or iron (II) sulfate with a concrete penetrant consisting of a suitable surfactant, or a solvent prepared by mixing sulfuric acid and iron oxide powders less than 1 μm in size with a concrete penetrant. When the solvent is coated in this manner on the surface of the concrete block 8, a penetrating layer 10 of iron sulfate or iron (II) sulfate or of sulfuric acid and iron oxide powders is formed on the concrete block surface, as in the case of the base plate 1 of Fig. 5, due to the assistive action of the surfactant (see Fig. 11 showing a portion D of Fig. 10 to an enlarged scale). The layer 10 acts to neutralize the alkali emitted from the concrete, as mentioned hereinabove.

When the concrete block is placed on the sea floor, diatoms or algae tend to be settled on the block due to the abundance of iron oxides and the aforementioned removal of the alkaline contents. Besides, sea urchins and shellfish swarm about the block to graze these diatoms or algae. In addition, the hemicircular recesses 9 provide the best habitation for them to find shelter from their enemies. It has been confirmed from the aforementioned experiment that the hemicircular recess 9 need be of a size to conform to the habitation of the sea urchins and shellfish and that the diameter of the recess approximately equal to 5 cm is preferred for the sea urchins or shellfish to survive and to defend their territory against invaders. The recess 9 need not be hemicircular but may also be of any other shape such as box-shaped provided that such shape of the recess has been found to be suitable for habitation of the aquatic animals.

As a result of the applicant's prolonged research on the habit and manner of living of sea urchins and shellfish, especially sea urchins, and the kinds of plants grazed by them, the artificial reef unit shown in Fig. 8 has been confirmed as satisfying certain basic culturing conditions. Thus, it has been found that abundant algae settle on the surface of the block 8 and each one recess 9 is occupied by one sea urchin. The recesses 9 on the lower side of the block 8 provide a good habitation for the young sea urchin and shellfish finding shelter from their enemies and are extremely useful to prevent depletion of sea urchins and shellfish.

Fig. 12 shows an artificial reef unit according to a modified embodiment of the present invention and especially designed for culturing abalone and top shells. Fig. 13 is a plan view of the reef unit and Fig. 14 is a sectional view taken along line E—E of Fig. 13. There is shown a multisided concrete block 12 providing the artificial reef unit of the present embodiment and presenting respective eight sides when seen in plan view and side elevation, the block 12 thus providing twenty-six flat sides. The block 12 is provided with numerous oblong recesses 13 on the upper five sides and the lower five sides as shown. These ten surfaces of the concrete block 12 are coated with a solvent consisting of a mixture of fine powders of iron sulfate or iron (II) sulfate with a concrete penetrant consisting in turn of a surfactant, or a solution consisting of a mixture of a concrete penetrant with sulfuric acid and fine powders of iron oxide less than 1 μm in diameter. When coated in this manner with the solvent, the surfaces of the concrete block 8 are formed with a penetrated layer 14 of iron sulfate or iron (II) sulfate or of acid and fine powders of iron oxides as in the case of the base plate 1 shown in Fig. 5 (see Fig. 15 showing a portion F of Fig. 14 to an enlarged scale) thus neutralizing the alkaline contents otherwise emitted from the concrete.

Besides, the layer 14 provides a good habitation for settlement of algae or diatom when the block is placed on the sea floor, in the same manner as described above in connection with the embodiment shown in Fig. 8.

In distinction from sea urchins, abalone and top shell, especially abalone, are extremely sensitive and escape from their habitation when the reef unit on which they rest are subjected to only slight vibration caused by the wave motion. It is therefore imperative that the reef unit be resting stably on the sea floor. The multisided body shown in Fig. 12 is preferred in point of stability to the block shown in Fig. 8 presenting a curved surface. Abalone and top shell are different in shape, although approximately equal in crust size. In general, abalone prefer to rest in recessed places on the rock surface, while the top shells tend to attach on the back side of the rock. Thus, when the multisided block 12 shown in Fig. 12 is placed on the surface of the sea floor, the top shells rest on the recesses 13 on the upper five surfaces, whereas abalone attach to the recesses 13 on the lower five surfaces. It has been found that the oblong recess 13 is preferably 8.5 cm in longer diameter, 5.0 cm in shorter diameter and about 5.0 cm in depth. The recesses need not be oblong but may be of any other shape such as box shape provided that such shape of the recesses is found to be suitable for the abalone and top shell to survive and defend their territory against invaders.

Figs. 16 to 19 illustrate a settlement reef unit adapted for settling and raising larval and juvenile fishes and shellfishes. Fig. 16 is a perspective view of a frame, Figs. 17 and 18 are front and side views, respectively, of a flocked plate and Fig. 19 is a front view of the reef unit. In the figures, the numeral 21 designates the frame comprised of a square bottom plate 22, four upright pillars 23 integrally formed with four corners of the bottom plate 22 and four girders 24 mounted across the upper ends of the girders 23. A central rib 25 is provided on the bottom plate 22 and a central rib 25' is also provided between the girders 24 in opposition to the lower rib 25. The bottom plate 22, the ribs 25, 25' and the girders 24 are formed with grooves generally indicated at 26.

A flocked plate 27 shown in the front view of Fig. 17 and in the side view of Fig. 18 is mounted in each of the grooves 26. On the sides of the flocked plate 27 except the side resting on the groove bottom, there are inserted pile yarns 28 consisting of natural or synthetic fiber. The plate 27 is placed in the groove 26 of the frame 21, a bolt 30 is passed through each through-hole 29 in the pillar 23 and a nut 31 tightened for securing the flocked plate 27 to the frame 21. In this manner, the plate 27 may be secured against accidental removal from the groove 26 due to wave motion or under the effect of oceanic currents. Four hoist hooks 32 are provided, one at each corner of the frame 21, for hoisting and submerging the reef unit with the aid of a crane or the like.

The raising or culturing reef unit comprised of the frame 21 and flocked plates 27 attached to the grooves 26 of the frame 21 is shown in assembled state in the front view of Fig. 19. When the raising reef unit is placed on the surface of the sea floor, the flocked plates 27 and the pile yarns 28 inserted on the plates protect larval and young fish and fishshell from their enemies, while the pile yarns 28 provide a habitation for settlement of the spores of sea weeds, plankton or the lower animals or plants and swimming organisms. Since a space is provided in the central zone where there are provided the ribs 25, 25', an upwardly directed turbulent flow is provided in the area with abundant supply of oxygen thus providing good habitation for the larval and young fish and shellfish.

Figs. 20 to 22 illustrate an example of a culturing reef unit for fish and shellfish, especially sea crayfish. Fig. 20 is a plan view of the reef unit. Fig. 21 a front view of the same and Fig. 22 a sectional view taken along line 20—20 of Fig. 20. In the drawing, the numeral 41 designates a generally flat plate-like concrete block presenting a rectangular shape when seen in plan view. The block 41 has a unitary flat lower surface 41a and an upper surface consisting of a flat central surface 41c and a tapered or inclined peripheral surface 41b. Therefore, the block 41 has a peripheral thickness $t_1$ lesser than the central thickness $t_2$ $(t_1 < t_2)$. The block 41 is cast integrally with six square-sided concrete pillars 41a, these pillars being arranged in two rows of three pillars and extending vertically from the lower surface 41a of the block 41. The upper surface of the block 41 is formed with six mating recesses for receiving the six pillars 41d. The numeral 42 designates a concrete block which is to be the base block of the reef unit and of a size slightly larger than two blocks 41 placed side by side. The support block 41 has twelve recesses for receiving the pillars 41d of the concrete blocks 41.

Two concrete blocks 41 are placed side by side on the base block 42 in a manner so that the pillars 41d on the lower surfaces 41a of the blocks 41 are received in the mating recesses on the upper surfaces of the base block 42. Four blocks 41 are stacked one on the other, with the pillars 41d on the lower surfaces 41a of the block 41 being received in the mating recesses on the upper surfaces of the subjacent block 41. Six bolts 43 are then passed from the bottom surface of the base block 12 and through openings in the blocks 41 stacked on the base block 42 and openings in the pillars 41d. Finally, nuts 44 are attached to the ends of the bolts and tightened for securing the base block 42 and the four stacked blocks 41 together.

The numeral 45 designates hooks by means of which the reef unit can be hoisted with a crane for placement thereof on the surface of the sea floor. Four hooks are provided one at each corner of the base block 42.

The distance from the lower surface 42a (see Fig. 22) of the base block 42 to the upper surface of the uppermost block 41 is preferably in the range of 1.5 to 2 m which is the usual range of activity or body movement of the sea crayfish. A central gap g2 between the adjoining blocks is selected in a manner so that the animal may hide itself against enemy and usually in the range of 7 to 30 cm depending on the animal size. A peripheral gap g1 between the adjoining blocks is selected in a manner so that the sea crayfish may enter therein without meeting substantial resistance.

In the above construction, the upper surface of the block 41 is comprised of the peripheral inclined surfaces 41b and the flat central surface 41c thus providing a streamlined profile for reducing the resistance presented to the sea water. The block 41 may have an increased central thickness t2 and an increased overall weight because the four-sided support pillars 41d are cast integrally with the concrete block, thus providing an extremely stabilized structure. The blocks 41 and the base blocks 42 are assembled to one another by six bolts 43 and nuts 44 with the pillars 41d received in the mating recesses thus providing a rigid structure resistant to vigorous wave motion usually encountered in the shallow sea zone.

Since the blocks 41 are made of concrete, an alkali contained in the concrete is emitted from the surface of the block 41 when the reef unit is submerged in the sea water. These alkaline contents are harmful to diatom, algae and sea animals and need be neutralized in the following manner.

First of all, when the mortar is cast into the frame, not shown, for forming the block 41, a large number of iron sulfate or iron (II) sulfate crystal blocks are embedded below the surface of the cast mortar while the mortar is not as yet cured. As the concrete is cured and dehydrated, the crystals 46 are dissolved in water from the concrete for forming a porous layer 47 presenting an irregular surface as shown in Fig. 24. This penetrating iron sulfate or iron (II) sulfate layer 47 is rugged on its front surface as shown in the enlarged view of Fig. 25 and penetrated on its reverse surface into the inside of the concrete like the roots of a tree so that it is not peeled off readily from the concrete surface. This iron sulfate or the iron (II) sulfate layer is rich in weakly acidic iron oxide contents because the alkali in the concrete is neutralized with acid from iron sulfate.

After the blocks 41 are hardened, the lower surface 41a of the block 41 is coated with a solvent consisting of a mixture of iron sulfate or iron (II) sulfate powders and a concrete penetrant consisting in turn of a surfactant. The iron sulfate or iron (II) sulfate powders are also penetrated from the surface into the inside of the concrete like the tree roots for providing a penetrating layer 48 as shown in an enlarged view of Fig. 27. It is preferred that the partial size of the iron sulfate or iron (II) sulfate powders be as small as possible.

The aforementioned solvent consisting of a mixture of iron sulfate or iron (II) sulfate powders and the concrete penetrant may be replaced by a solvent consisting of fine powders of iron oxide such as $\lambda$-iron oxide less than 1 $\mu$m in particle size, a small amount of sulfuric acid and a concrete penetrant consisting in turn essentially of surfactant. With the use of this solvent, sulfuric acid and iron oxide powders are penetrated from the surface into the inside of the concrete like tree roots due to the assistive action of the surfactant, thus providing a penetrated layer 48 similar to the one shown in Figs. 26 and 27.

The layer 48 is weakly acidic because the strong alkali (with pH approximately 13) contained in the concrete block 41 is neutralized with sulfuric acid in the solvent or with the acid yielded upon dissolution of iron sulfate or iron (II) sulfate.

With the use of the reef unit consisting of the base block 12 and the blocks 41 having the porous penetrated layer 47 derived from the iron sulfate or iron (II) sulfate crystals on the upper surface and the penetrating layer 48 derived from the solvent on the reverse surface, no alkalis are ejected from the surface of the block 41, while there is an abundant supply of iron ions liked by sea crayfish.

Instead of providing the penetrated layer 47 derived from the iron sulfate or iron (II) sulfate powders and the layer 48 derived from the solvent on the upper and lower surfaces of the blocks 41, the penetrated layers 47, 48 may be provided on the reverse and front surfaces of the blocks, respectively. Alternatively, both surfaces of the block may be provided with porous layers 47 or with penetrated layers 48 from the solvent.

In the above embodiment, only one or three or more rows or concrete blocks 41 each consisting of plural tiers of blocks 41 may be mounted on the base block. The tapered peripheral surfaces may be provided to the lower peripheral surface or to both the upper and lower peripheral surfaces of the concrete block.

Figs. 28, 29 show an artificial culturing and fishing area unit for culturing fish and fishshell, especially sea crayfish, which is combined from the artificial reef unit shown in Fig. 12, the settlement reef unit shown in Figs. 16 to 19 and the raising or culturing reef unit shown in Figs. 20, 21. Fig. 28 shows the artificial fishing area unit. Fig. 28 is a front view and Fig. 29 a plan view of the unit. In the figure, the numerals 51, 52 designate settlement reef units similar to those shown in Figs. 16 to 19 and on which larval or juvenile fishes and fishshells are settled. These reef units are arranged side by side on a bed of natural pebbles. On both sides of and adjacent to these two reef units 51, 52, plural reef blocks 12 as shown in Fig. 8 are stacked for forming block partitions 54, 55. To the outside of these block partitions 54, 55 are placed raising reef blocks 56, 57 for raising the sea crayfish as shown in Figs. 20, 21. Natural pebbles are placed in the interstices between the reef blocks 51, 52 and the interstices between the block partitions 54, 55 for providing natural pebble partitions 59, 60. Natural pebbles are also stacked in the interstice between the raising reef block 56 and the block partition 54 and in the interstice between the raising reef block 57 and the block partition 55. Reef blocks 12 shown in Fig. 12 are placed around the raising reef blocks 56, 57 in block rows 62 to 67.

With the use of the reef blocks 12 each weighing about 500 kg and the block partitions 54, 55 each weighing about 18 tons, settlement reef blocks 51, 52 can be fixed stably.

The sea water flows into the reef in the direction shown by arrow marks I or J in Fig. 29 under the effect of wave motion or oceanic currents, thus producing an upwardly directed turbulent flow at the central zone. Thus the fish and shellfish, especially the larval and young sea crayfish living in the sea water are affixed to and settled on settlement blocks 51, 52. The larval or young sea crayfish feed on diatoms and plant or animal plankton deposited on settlement blocks 51, 52, block partitions 54, 55 on both sides of the blocks 51, 52 and the partitions 58 to 61 of natural pebbles.

It is known that sea crayfish are fond of iron ions because they find their habitation near the sunken vessel or on the rock abundant in ferrous contents. The sea crayfish are nocturnal animals and forage for food during night but hide themselves under the pebbles or between the rocks during daytime. Their favorite foods are shellfishes. As sea crayfish grow in size, they change their habitat from settlement blocks 51, 52 to the raising blocks 56, 57. The interstices g2 between the blocks 41, 41 shown in Figs. 20 and 21 constituting the settlement blocks 56, 57 are equal to the gap between the rocks in which the sea crayfish rest during daytime, and provide a place where there is abundant supply of ferrous ions liked by sea crayfish. Thus they move to the raising blocks 56, 57 from settlement blocks 51, 52 as the animals grow to the adult stage.

In addition, sea weeds of various kinds grow on the rows 62 to 67 of the reef blocks 12 provided about the raising blocks 56, 57 so that abalone top shell and other shellfish grazing these sea weeds find their habitation about these blocks 12, the sea crayfish in turn foraging on these shellfishes. Hence, the artificial fishing area unit represents a culturing zone best suited for the sea crayfish.

Fig. 30 shows an example of a combined artificial fishing area consisting of a plurality of artificial fishing area units described hereinabove. In the figure, the combined artifical fishing area unit is composed of seven fishing area units B1 to B7 with each unit B1 to B7 consisting of settlement blocks 51, 52 and raising blocks 56, 57 as shown in Fig. 29. The unit B1 is arranged with respect to the units B2, B3 in a manner so that settlement blocks 51, 52 of the unit B1 are located close to the raising blocks 56, 57 of the units B2, B3. Similarly, the unit B4 is placed with respect to the units B2, B3, B5 and B6 in a manner so that settlement blocks 51, 52 of the unit B4 are located close to the raising units 56, 57 of the units B2, B3, B5 and B6, while the unit B7 is placed with respect to the units B5, B6 in a manner so that settlement blocks 51, 52 of the unit B7 are located close to the raising blocks 56, 57 of the units B5, B6. By thus combining the units B1 to B7, there is provided an extensive artificial fishing area unit in which sea crayfish may defend themselves against enemy and find sufficient food in the course of their lifetime since hatching from larvae until they grow to adults through the larval and juvenile stages.

In the above embodiment, the artificial fishing area unit shown in Figs. 29 or 30 is composed of settlement reef units shown in Figs. 16 to 19, the raising reef units shown in Figs. 20, 21 and artificial reef units shown in Fig. 12, but this is not essential.

**Claims**

1. A concrete block suitable for use underwater as a reef for the growth of algae, characterised in that the block (1) is provided on its surface with an iron sulfate in a form which neutralises alkali leached from the concrete.

2. A block as claimed in Claim 1, wherein the iron sulfate is present discontinuously in the form of crystals (4) embedded at least partially in recesses (2) in the surface of the block before the concrete was hardened whereby the parts of the crystals in contact with the concrete dissolve in the water content of the concrete as the concrete hardens to thereby penetrate the concrete surface of the recesses.

3. A block as claimed in Claim 1, wherein the iron sulfate is present in a layer (5) on at least one surface of the block (1), the layer (5) being formed by coating a mixture of particles of the iron sulfate, or of iron oxide and sulfuric acid, and a liquid containing a surfactant onto said surface so that the particles penetrate the surface of the concrete.

4. A block as claimed in any preceding claim, which has surface recesses (9, 13) of a size and shape suitable for habitation by edible sea animals.

5. A block (8, 12) as claimed in any preceding claim, having a plurality of curved or flat surfaces and which is suitable by itself for use as an underwater reef.

6. An assembly for use as an artificial reef unit, which comprises a plurality of blocks as claimed in Claim 1, 2, 3 or 4, fastened together to form a structure (6) stable underwater and each having its iron sulfate bearing surface directed outwards so that algae can grow thereon.

7. An assembly as claimed in Claim 6, which comprises a plurality of plate-like concrete blocks (41) each having said iron sulfate on one or both surfaces, the blocks being stacked in one or more tiers and secured to a base block (41), the blocks in the tiers being separated by gaps of a size to allow a sea crayfish or similar animal to creep thereinto.

8. An assembly as claimed in Claim 7, wherein the upper surface of the blocks has crystals of an iron sulfate crystal embedded below its surface and the lower surface is coated with a mixture of powdered iron sulfate or sulfuric acid and iron oxide powder, and a surfactant.

9. An artificial culturing and fishing unit, comprising a combination of different types of concrete blocks or assemblies as claimed in any preceding claim and suitable for the growth of fish or edible underwater animals of different types.

## Patentansprüche

1. Zur Verwendung unter Wasser als Riff zum Algenwachstum geeigneter Betonblock, dadurch gekennzeichnet, dass der Block (1) auf seiner Oberfläche mit einem Eisensulfat in einer Form versehen ist, die aus dem Beton ausgelaugtes Alkali neutralisiert.

2. Block nach Anspruch 1, worin das Eisensulfat diskontinuierlich in Form von Kristallen (4) vorliegt, die zumindest teilweise vor dem Abbinden des Betons in Ausnehmungen (2) in der Blockoberfläche eingebettet wurden, so dass sich die mit dem Beton in Berührung stehenden Teile der Kristalle während des Abbindens des Betons in dessen Wassergehalt auflösen und dadurch in die Betonoberflächen der Ausnehmungen eindringen.

3. Block nach Anspruch 1, worin das Eisensulfat in einer Schicht (5) auf mindestens einer Oberfläche des Blocks (1) vorliegt, wobei die Schicht (5) durch Beschichtung dieser Oberfläche mit einem Gemisch von Teilchen aus Eisensulfat oder Eisenoxyd und Schwefelsäure sowie einer ein Tensid enthaltenden Flüssigkeit gebildet wird, so dass die Teilchen in die Betonoberfläche eindringen.

4. Block nach einem der vorhergehenden Ansprüche, welcher Oberflächenausnehmungen (9, 13) einer zur Ansiedlung von essbaren Meerestieren geeigneten Grösse und Gestalt aufweist.

5. Block (8, 12) nach einem der vorhergehenden Ansprüche mit einer Mehrzahl gekrümmter oder flacher Oberflächen, der an sich zur Verwendung als Unterwasserriff geeignet ist.

6. Aufbau zur Verwendung als künstliche Riffeinheit, bestehend aus einer Mehrzahl von Blöcken nach Anspruch 1, 2, 3 oder 4, die zur Bildung einer unter Wasser stabilen Konstruktion (6) zusammengefügt sind und deren Eisensulfat tragende Oberflächen jeweils nach aussen gerichtet sind, so dass Algen darauf wachsen können.

7. Aufbau nach Anspruch 6, bestehend aus einer Mehrzahl plattenartiger Betonblöcke (41), die je Eisensulfat auf einer oder beiden Oberflächen aufweisen, wobei die Blöcke in einer oder mehreren Lagen gestapelt und an einem Grundblock (41) befestigt sind, wobei die Blöcke in den Lagen durch Spalten einer Grösse getrennt sind, die es einem Seekrebs und ähnlichen Tieren ermöglichen, dort hineinzukriechen.

8. Aufbau nach Anspruch 7, worin die obere Fläche der Blöcke darunter eingebettete Eisensulfatkristalle aufweist und die untere Fläche mit einem Gemisch aus gepulvertem Eisensulfat oder Schwefelsäure und Eisenoxydpulver sowie einem Tensid beschichtet ist.

9. Künstliche Kultur- und Fischzuchteinheit, bestehend aus einer Kombination verschiedener Arten von Betonblöcken oder Aufbauten nach einem der vorhergehenden Ansprüche, geeignet für das Wachstum von Fischen oder essbaren Unterwassertieren verschiedener Arten.

## Revendications

1. Un bloc de béton convenable pour l'emploi subaquatique en tant que récif pour la croissance des algues, caractérisé en ce que le bloc (1) est pourvu à sa surface d'un sulfate de fer sous une forme qui neutralise les alcalis lessivés du béton.

2. Un bloc selon la revendication 1, dans lequel le sulfate de fer est présent de manière discontinue sous forme de cristaux (4) incrustés au moins partiellement dans des cavités superficielles du bloc avant que le béton (2) ne durcisse, ce par quoi les parties des cristaux en contact avec le béton se dissolvent dans l'eau que contient le béton pendant que le béton durcit pour pénétrer par là dans la surface de béton des cavités.

3. Un bloc selon la revendication 1, dans lequel le sulfate de fer est présent dans une couche (5) sur au moins une face du bloc (1), la couche (5) étant formée en enduisant un mélange de particules du sulfate de fer, ou d'oxyde de fer et d'acide sulfurique et d'un liquide contenant un agent tensio-actif sur ladite face de manière à ce que les particules pénètrent dans la surface du béton.

4. Un bloc selon l'une quelconque des revendications précédentes, qui a des cavités superficielles (9, 13) de dimensions et de forme convenables pour l'habitation par des animaux marins comestibles.

5. Un bloc (8, 12) selon l'une quelconque des revendications précédentes ayant une pluralité de faces arrondies ou plates et qui est convenable en soit pour un usage en tant que récif sub-aquatique.

6. Un assemblage destiné à un emploi en tant que groupe de récif artificiel qui comprend une pluralité de blocs selon la revendication 1, 2, 3 ou 4 adaptés ensemble pour former une structure (6) stable sous l'eau et ayant chacun sa face porteuse de sulfate de fer dirigée vers l'extérieur de manière à ce que des algues puissent croître dessus.

7. Un assemblage selon la revendication 6, qui comprend une pluralité de blocs de béton en forme de plaques (41) chacun ayant ledit sulfate de fer sur une face ou les deux faces, les blocs étant empilés sur une ou plusieurs rangées et fixés à un bloc de base (41), les blocs dans les rangées étant séparés par des espaces d'une dimension suffisante pour permettre à une langouste ou à un animal similaire de passer à travers.

8. Un assemblage selon la revendication 7, dans lequel la face supérieure des blocs comporte des cristaux d'un cristal de sulfate de fer incrustés sous sa face et la face inférieure est enduite d'un mélange de sulfate de fer pulvérisé ou d'acide sulfurique et de poudre d'oxyde de fer et d'un agent tensio-actif.

9. Une unité de pêche et de culture artificielle, comprenant une combinaison de différents types d'assemblages ou de blocs de béton selon l'une quelconque des revendications précédentes et convenables pour la croissance de poissons ou d'animaux sub-aquatiques comestibles de différents types.

# Fig. 1

# Fig. 2

# Fig. 3

# Fig. 4

## Fig.5

## Fig.6

## Fig.7

Fig. 8

Fig. 9

Fig. 10

Fig. 11

# Fig. 12

# Fig. 13

# Fig. 14

Fig.15

Fig.16

Fig.17

Fig.18

Fig.19

Fig.20

6

Fig.21

Fig.22

Fig. 23

Fig. 24

Fig. 25

Fig.26

Fig.27

Fig.28

0 134 855

# Fig. 29

0 134 855

# Fig.30